# EUROPEAN PATENT APPLICATION

(11) **EP 4 772 125 A1**
(43) Date of publication of application: **08.07.2026**
(21) Application number: 25217868.6
(22) Date of filing: 24.11.2025
(51) Int. Cl.: A61B 18/14

(54) **MEDICAL PROBE INCLUDING A GENERALLY SEMI-SPHERICAL OR SEMI-CONICAL MEMBRANE**

(30) Priority: 31.12.2024 US 202419006934
(71) Applicant: Biosense Webster (Israel) Ltd., 2066717 Yokneam (IL)
(72) Inventor: VAN NIEKERK, Pieter Emmelius, Irvine, 92618 (US); SUAREZ, Paul, Irvine, 92618 (US); HIGHSMITH, Debby, Irvine, 92618 (US); BASU, Shubhayu, Irvine, 92618 (US); HENRIQUEZ, Jamie Lynn, Irvine, 92618 (US); BAR-TAL, Meir, 2066717 Yokneam (IL); BERGER, Abraham, 2066717 Yokneam (IL); BERGER, Omer, 2066717 Yokneam (IL)
(74) Representative: Carpmaels & Ransford LLP

(57) **Abstract**

The disclosed technology includes a distal tip of a medical probe. The distal tip includes spines extending radially outward from a longitudinal axis of the end effector in an expanded configuration, a membrane connected to the spines, a first flexible circuit disposed on a first surface of the membrane proximate each of the plurality of spines, a second flexible circuit disposed on a second surface of the membrane proximate each of the plurality of spines, a plurality of first pairs of electrodes disposed on the first flexible circuit proximal to one another and a spine of the plurality of spines, and a plurality of second pairs of electrodes disposed on the second flexible circuit proximal to one another and a spine of the plurality of spines such that the first and second pairs of electrodes define a mirror image configuration.

## Description

### FIELD

The present technology relates generally to medical devices, and in particular medical probes with electrodes, and further relates to, but not exclusively, medical probes suitable for use to map or ablate tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0196372A1, 2021/0177503A1, and 2021/0186604A1, and U.S. Pat. No. 11,540,877 each of which are incorporated herein by reference in their entireties and attached in the Appendix hereto.

Regions of cardiac tissue can be mapped by a catheter to identify the abnormal electrical signals. Some catheter ablation procedures especially those with persistent atrial fibrillation may be performed using electrophysiology (EP) mapping to target areas of aberrant electrical signals. Such EP mapping may include the use of diagnostic electrodes configured to monitor electrical signals within the cardiovascular system to pinpoint the location of aberrant conductive tissue sites that are responsible for the arrhythmia. Examples of an EP mapping system are described in U.S. Patent No. 5,738,096, incorporated herein in its entirety by reference. Examples of EP mapping catheters are described in U.S. Patent No. 9,907,480, U.S. Patent Pub. No. 2018/0036078, and U.S. Patent Pub. No. 2018/0056038, each of which are incorporated herein by reference in their entireties.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, Calif.

In current practice, the effectiveness of the delivery of IRE energy is dependent on the skill of the physician, meaning the patient can suffer from incomplete isolation of target areas. In order to effectively deliver the IRE energy to ablate, ablation catheters typically need to be reoriented multiple times during a procedure, which increases procedure time as well as complicates the ablation process. Moreover, existing catheters generally require stiff internal structural members to ensure that a predetermined configuration is maintained. The stiffness is a disadvantage during manipulation in the body organ as it can prevent electrodes from contacting the tissue. Other catheters can include flexible end effectors designed to overcome this disadvantage. These catheters can include layered components that can be timeconsuming, complex, and expensive to manufacture and assemble. Accordingly, there is a need for an improved end effector of a medical probe that addresses these problems that is capable of ablating and mapping.

### SUMMARY

There is provided, in accordance with the disclosed technology, an end effector of a medical probe, the end effector comprising: a plurality of spines extending radially outward from a longitudinal axis of the end effector in an expanded configuration; a membrane connected to the plurality of spines, the membrane comprising a distal end that surrounds the longitudinal axis and defining a volume with an open end, the membrane including a first surface and a second surface opposite the first surface; a first flexible circuit disposed on the first surface of the membrane proximate each of the plurality of spines and extending around the longitudinal axis; a second flexible circuit disposed on the second surface of the membrane proximate each of the plurality of spines and extending around the longitudinal axis; a plurality of first pairs of electrodes disposed on the first flexible circuit proximal to one another and a spine of the plurality of spines; and a plurality of second pairs of electrodes disposed on the second flexible circuit proximal to one another and a spine of the plurality of spines such that the first and second pairs of electrodes, with the membrane between the first and second pairs of electrodes, define a mirror image configuration.

There is further provided, in accordance with the disclosed technology, a medical system comprising: a medical probe comprising an elongated probe body and an end effector connected to a distal end of the elongated probe body, the elongated probe body and the end effector extending along a longitudinal axis, the end effector comprising: a plurality of spines connected to the elongated probe body and extending radially outward from the longitudinal axis of the end effector in an expanded configuration; a membrane connected to the plurality of spines, the membrane comprising a distal end that surrounds the longitudinal axis and defining a volume with an open end, the membrane including a first surface and a second surface opposite the first surface; a first flexible circuit disposed on the first surface of the membrane proximate each of the plurality of spines and extending around the longitudinal axis; a second flexible circuit disposed on the second surface of the membrane proximate each of the plurality of spines and extending around the longitudinal axis; a plurality of first pairs of ablation electrodes disposed on the first flexible circuit and proximal to one another and a spine of the plurality of spine; and a plurality of second pairs of electrodes disposed on the second flexible circuit proximal to one another and a spine of the plurality of spines such that the first and second pairs of electrodes, with the membrane between the first and second pairs of electrodes, define a mirror image configuration; and an ablation generator configured to provide ablation pulses to the plurality of first pairs of electrodes and the plurality of second pairs of electrodes.

There is further provided, in accordance with the disclosed technology, a method of manufacturing an end effector for a medical probe, the method comprising: forming a plurality of spines extending away from a longitudinal axis; disposing a first flexible circuit on a first side of the plurality of spines, the first flexible circuit comprising a first flexible substrate and a first pair of electrodes; disposing a second flexible circuit on a second side of the plurality of spines, the second flexible circuit comprising a second flexible substrate and a second pair of electrodes; placing a first sheet of insulative material in contact with the first flexible circuit, the first sheet surrounding the longitudinal axis; placing a second sheet of insulative material in contact with the second flexible circuit, the second sheet surrounding the longitudinal axis and defining a volume with an open end; and molding the first sheet and the second sheet to envelop the first flexible circuit and the second flexible circuit.

### BRIEF DESCRIPTION OF THE DRAWINGS

FIG. 1 is a schematic pictorial illustration of a medical system including a medical device that includes a medical probe with a distal tip with electrodes, in accordance with the disclosed technology;
FIG. 2A is a schematic pictorial illustration showing a perspective view of a distal side of a distal tip of the medical probe in an expanded configuration, in accordance with the disclosed technology;
FIG. 2B is a schematic pictorial illustration showing a perspective view of a distal side of an alternative configuration of the distal tip of the medical probe in an expanded configuration, in accordance with the disclosed technology;
FIG. 3 is a schematic pictorial illustration showing a perspective view of a proximal side of the distal tip of the medical probe of FIG. 2A in the expanded configuration, in accordance with the disclosed technology;
FIG. 4A is a schematic pictorial illustration showing a cross-sectional view, taken along line 4A-4A in FIG. 2A, of the distal tip, in accordance with the disclosed technology;
FIG. 4B is a schematic pictorial illustration showing a cross-sectional view, similar to that of FIG. 4A, of the alternative configuration of the distal tip of FIG. 2B, in accordance with the disclosed technology;
FIG. 5A is a schematic pictorial illustration showing a detail view of Detail A in FIG. 4A, showing an exemplary ablation configuration of the electrodes, in accordance with the disclosed technology;
FIG. 5B is a schematic pictorial illustration showing a similar detail view as that of FIG. 5A, showing an alternative ablation configuration of the electrodes, in accordance with the disclosed technology;
FIG. 5C is a schematic pictorial illustration showing a similar detail view as that of FIG. 5A, showing an alternative ablation configuration of the electrodes, in accordance with the disclosed technology;
FIG. 6 is a schematic pictorial illustration showing a similar detail view of FIG. 5A, showing an alternative configuration of the electrodes, in accordance with the disclosed technology;
FIG. 7 is a schematic pictorial illustration showing a similar detail view of FIG. 5A, showing an alternative configuration of the membrane, in accordance with the disclosed technology;
FIG. 8 is a schematic pictorial illustration showing a similar detail view of FIG. 5A, showing an alternative configuration of the membrane, in accordance with the disclosed technology;
FIG. 9 is a schematic pictorial illustration showing a perspective view of a distal side of another distal tip in an expanded configuration, in accordance with the disclosed technology;
FIG. 10 is a schematic pictorial illustration showing a cross-sectional view, taken along line 10-10 in FIG. 9, of another distal tip, in accordance with the disclosed technology;
FIG. 11 is a schematic pictorial illustration showing a perspective view of a distal side of another exemplary distal tip in an expanded configuration, in accordance with the disclosed technology;
FIG. 12 is a schematic pictorial illustration showing a side view of the distal tip of FIG. 11 disposed in a sheath, in accordance with the disclosed technology;
FIG. 13 is a flow chart depicting a method of manufacturing a distal tip of a medical probe, in accordance with the disclosed technology;
FIG. 14 is a schematic pictorial illustration showing a first example electrode configuration for the example end effector including elongated ablation electrodes and diagnostic electrodes positioned alongside the ablation electrodes, in accordance with the disclosed technology;
FIG. 15 is a schematic pictorial illustration showing a second example electrode configuration for the example end effector including elongated ablation electrodes which are respectively segmented into small parallel stripes and diagnostic electrodes positioned alongside the ablation electrodes, in accordance with the disclosed technology;
FIG. 16 is a schematic pictorial illustration showing a third example electrode configuration for the example end effector including rectangular elongated ablation electrodes without diagnostic electrodes, in accordance with the disclosed technology; and
FIG. 17 is a schematic pictorial illustration showing a fourth example electrode configuration for the example end effector including rectangular elongated ablation electrodes interrupted by diagnostic electrodes, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positivevoltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Each phase of the biphasic and monophasic pulse preferably has a square shape having an essentially constant voltage amplitude during a majority of the phase duration. Phases of the biphasic pulse may be separated in time by an interphase delay.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, methods, uses, and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

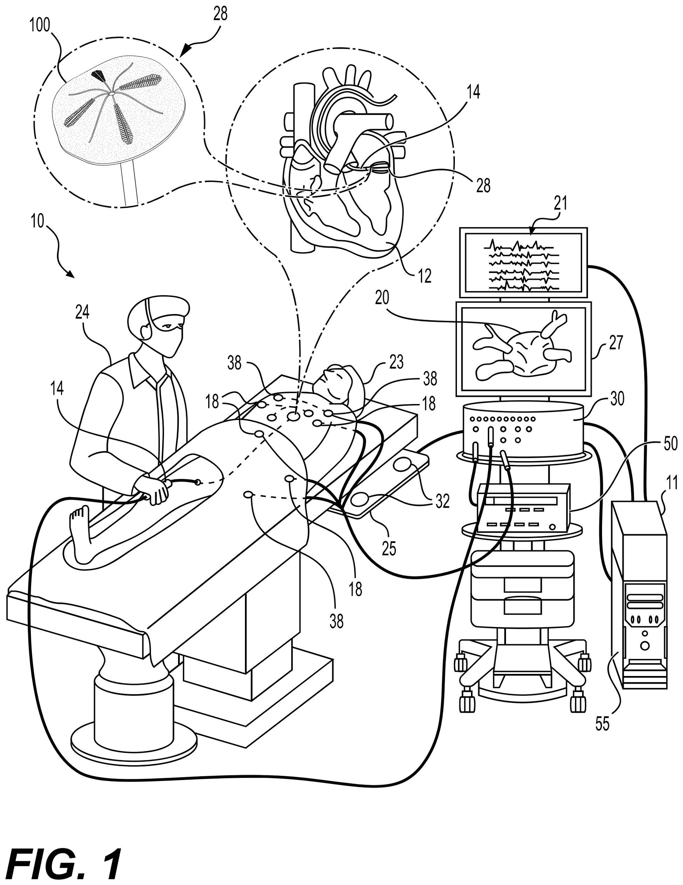

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating or catheters dedicated for both sensing and ablating. An example catheter/medical probe 14 that is configured for sensing IEGM is illustrated herein. Physician 24 brings a catheter shaft with a distal tip 28 (*e.g.,* a multi-layered end effector 100) of catheter 14 into contact with the heart wall for sensing a target site in heart 12. For ablation, physician 24 would similarly bring a distal end of an ablation catheter to a target site for ablating.

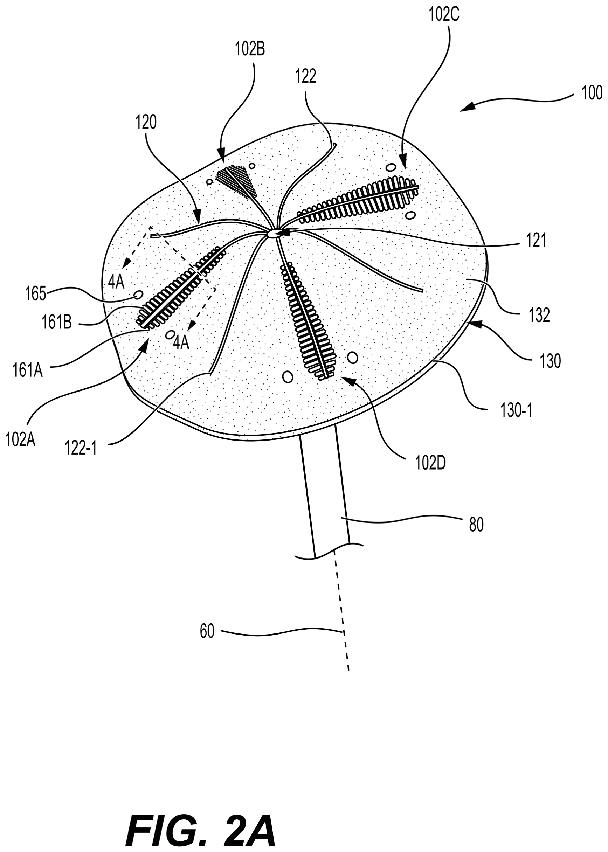

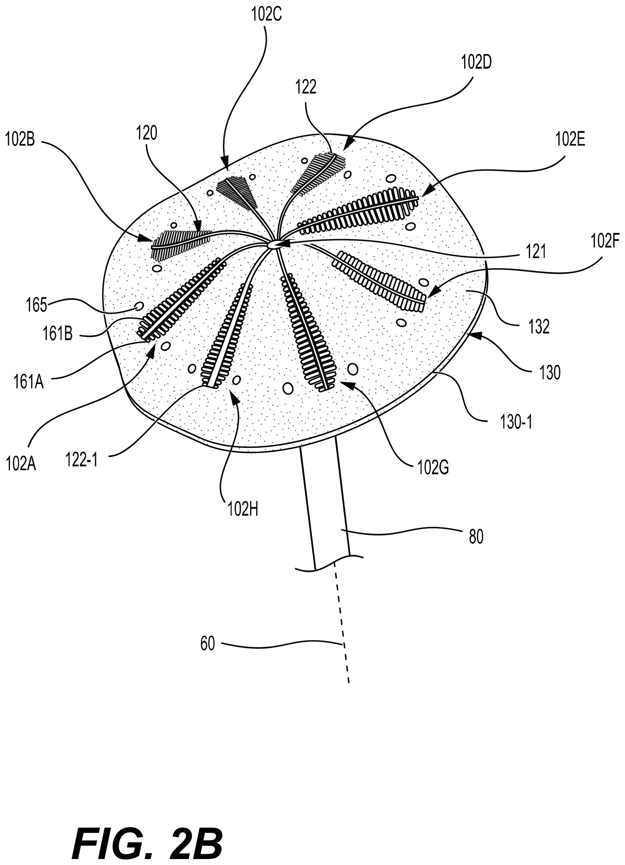

Catheter 14 is an exemplary catheter that includes multiple electrodes 160 (see FIGs. 2A and 2B), which include ablation electrodes 161 and diagnostic electrodes 165. The diagnostic electrodes 165 are positioned proximal to or are surrounded by the ablation electrodes 161 and are configured to sense the IEGM signals and to aid in confirming contact with tissue. In examples described herein, electrodes 160 can be configured to deliver ablation energy (IRE or RF) to tissue in heart 12. In addition to using electrodes 160 to deliver ablation energy, the electrodes 160 can also be used to determine the location of the end effector 100 or to measure a physiological property such as local surface electrical potentials at respective locations on tissue in heart 12. The electrodes 160 can be biased such that a greater portion of the electrode 160 faces outwardly from the end effector 100 such that the electrodes 160 deliver a greater amount of electrical energy outwardly away from the end effector 100 (i.e., toward the heart 12 tissue) than inwardly toward the end effector 100.

Examples of materials ideally suited for forming electrodes 160 (which include electrodes 161 and 165) include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes 160 on the inner sides of the spines), and then to the blood pool in heart 12. Additionally, silver epoxy/inks can be employed, which can be used to increase surface area to reduce impedance and improve flexibility. In some examples, impedance reducing coatings, such as iridium oxide (IrOx) or a platinum-iridium (PtIr) alloy can be employed.

Catheter 14 may additionally include a position sensor embedded in or near distal tip 28 for tracking position and orientation of distal tip 28. Optionally and preferably, the position sensor is a magnetic based position sensor including three magnetic coils for sensing three-dimensional (3D) position and orientation.

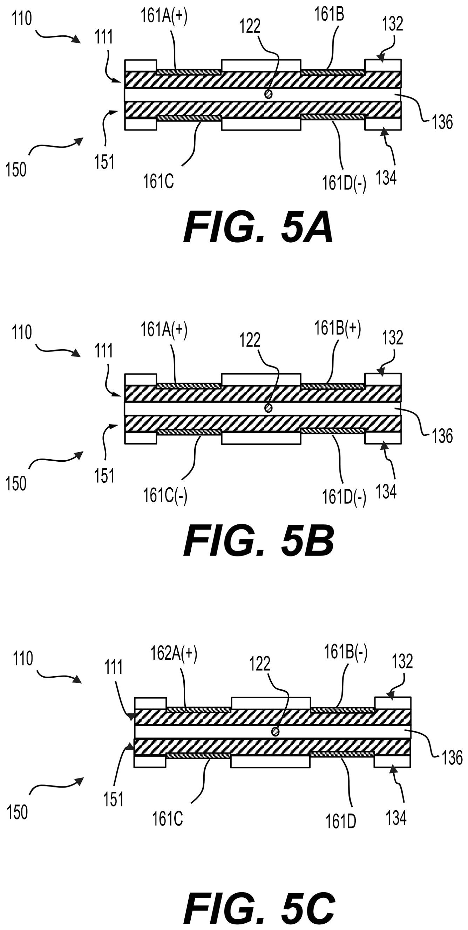

Magnetic based position sensor may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference. The end effector 100 may further include one or more inductive coils configured to provide electrical signals to the magnetic based position sensing system to determine location or orientation of the end effector. For instance, the end effector 100 may include inductive loops or coils similar to as illustrated in Figures 5A and 5B of U.S. Patent Publication No. 2024/0215894 incorporated by reference in its entirety herein and attached in the Appendix hereto. In some examples, one or more inductive coils in the end effector 100 may be used together with the position sensor to determine location or orientation of the end effector 100.

System 10 includes one or more electrode patches 38 positioned for skin contact on patient 23 to establish location reference for location pad 25 as well as impedance-based tracking of electrodes 160. For impedance-based tracking, electrical current is directed toward electrodes 160 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 160 of catheter 14. Recorder 11 may include pacing capability for pacing the heart rhythm or may be electrically connected to a standalone pacer.

System 10 may include an ablation energy generator 50 that is adapted to conduct ablative energy to one or more of electrodes 160 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage direct current (DC) or alternating current (AC) pulses as may be used to effect irreversible electroporation (IRE), or combinations thereof. The ablation energy generator 50 is preferably configured to provide biphasic bipolar pulses to induce IRE while keeping tissue temperature below thermal ablation temperatures. Additionally, or alternatively the ablation energy generator 50 is configured to provide monophasic IRE pulses, unipolar IRE pulses, thermal ablation electrical signals, or combinations thereof. For instance, the ablation energy generator 50 can be configured to provide pulses similar to as described in in U.S. Patent Pub. No. 2021/0169550A1, 2021/0177503A1, 2021/0186604A1, and 2023/0009191A1 and U.S. Patent No. 11,540,877B2, each of which are incorporated herein by reference in their entireties and attached in the Appendix hereto. U.S. Patent No. 11,540,877B2 corresponds to U.S. Patent Pub. No. 2021/0161592A1, which is incorporated here by reference in its entirety.

For instance, as described in U.S. Patent No. 11,540,877B2, the generator 50 can be configured to apply bipolar pulses having an amplitude sufficient to cause IRE in the tissue contacted by the electrodes and also RF energy having power sufficient to thermally ablate the tissue contacted by the electrodes. In some embodiments, the sequence of bipolar pulses includes pulses having an amplitude of at least 200 V, and a duration of each of the bipolar pulses is less than 20 µs. Additionally, or alternatively, the RF signal has a frequency between 350 and 500 kHz and an amplitude between 10 and 200 V. The end effector may further include temperature sensors and the electrical signal generator can be configured to apply the signals responsively to a temperature measured by the temperature sensors. In some embodiments, the IRE signal may have parameters as indicated in Table 1 of U.S. Patent No. 11,540,877B2. Note that the "bipolar pulse" as described in U.S. Patent No. 11,540,877B2 is referred as a "biphasic pulse" herein which relates to the shape of an electrical signal; whereas a "bipolar pulse" as described herein relates to the arrangement of electrodes receiving the pulse as defined herein above.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations. The PIU 30 can control the generator 50 to provide electrical energy to the ablation electrodes of the end effector 100 according to the ablation protocols described above and in the above incorporated references. The PIU 30, workstation 55, and the generator 50 can collectively be considered an ablation system console having one or more output ports configured to provide ablation energy to the ablation electrodes, one or more processors, and non-transitory computerreadable medium in communication with the processor to cause the ablation system console to provide ablation energy as described above and in the examples herein below.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

In some examples, the system 10 further includes an irrigation system configured to irrigate during IRE. In some embodiments, the PIU 30 is configured to control the irrigation system to provide irrigation to the catheter end effector similar to as described in U.S. Patent Pub. No. 2021/0196372A1 incorporated by reference in its entirety herein and attached in the Appendix hereto. The irrigation fluid may exit the distal end of the catheter 14 through a port at the distal end of the shaft 80 or through pores in the body of the end effector 100.

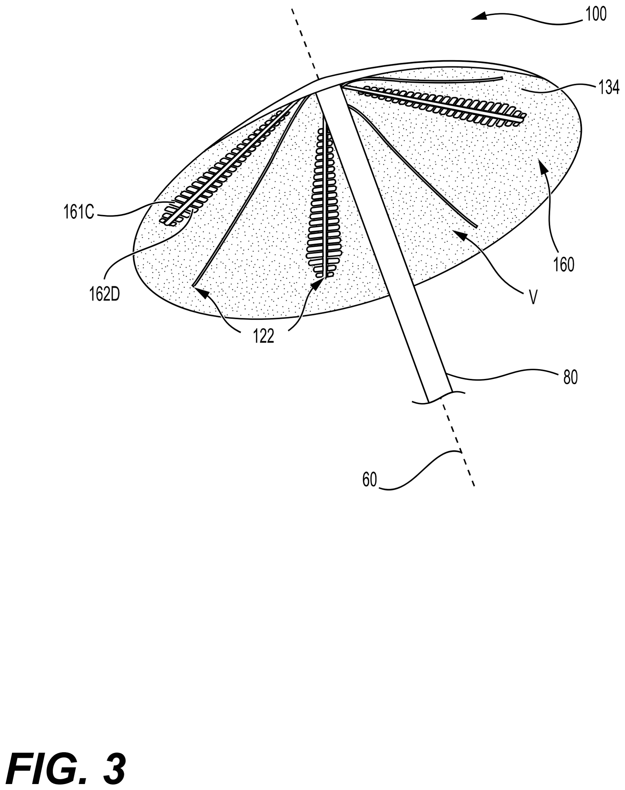

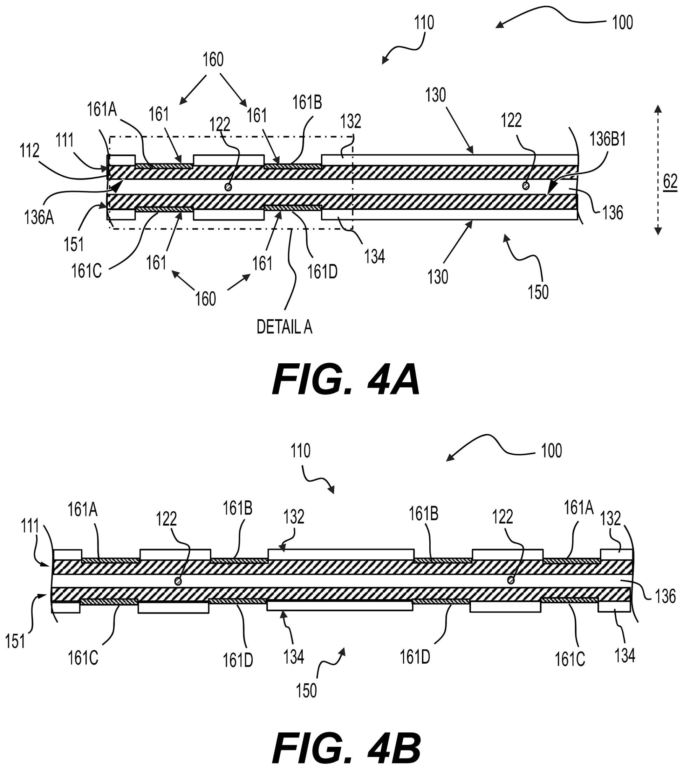

FIG. 2A is a schematic pictorial illustration showing a perspective view of a distal side of a distal tip 28 of the catheter 14. FIG. 3 is a schematic pictorial illustration showing a perspective view of a proximal side of the distal tip 28 of the catheter 14. FIG. 4A is a schematic pictorial illustration of a cross-sectional view of a portion of the distal tip 28.

It is noted that FIG. 4A, as well as the other cross-sectional figures in the present application, are depicted with the illustrated portion of the end effector 100 being flat/planar. However, those skilled in the art will appreciate that these illustrations are merely intended to relay, to the reader, potential configurations of the electrodes 160 or layers of the end effector 100. In use, the end effector has a shaped similar to an umbrella (discussed in greater detail below); therefore, a more technically accurate depiction of cross-sectional view of the end effector 100 would either be approximately arcuate in form or would angularly redirect at the spines 122 (discussed in greater detail below) of the end effector 100.

Making reference now to FIGs. 2A and 3, a distal tip 28 (FIG. 1) of the catheter/medical probe 14 includes an end effector 100 extending from an elongated probe shaft 80 and along a longitudinal axis 60. In some examples, the probe shaft 80 carries the aforementioned irrigation system. The end effector 100 includes a spine framework 120 that extends along the longitudinal axis 60.

The framework 120 includes an attachment section 121 connected to the probe shaft 80 and a plurality of spines 122 extending radially outwardly from the attachment section 121 and longitudinal axis 60. In the example of FIGs. 2A and 3, each spine has a fixed end (at the attachment section 121) and a free distal end 122-1. In the present example, eight spines 122 are employed are angularly/radially disposed about the longitudinal axis 60. However, any appropriate number of spines (i.e., at least three) can be employed without departing from the spirit and scope of the present disclosure, provided that they are capable of supporting a three-dimensional structure membrane 130 (discussed in greater detail below).

In some examples, the framework 120 is unitary (i.e., a monolithic structure). In such examples, the framework 120 can be formed from a planar or cylindrical tube stock of material using any suitable method. For example, the framework 120 can be formed by cutting, laser cutting, stamping, combinations thereof, etc. such that it is split to form the spines 122 and define the attachment section 121. In other examples, the spines 122 can be discrete members that converge at the attachment section 121.

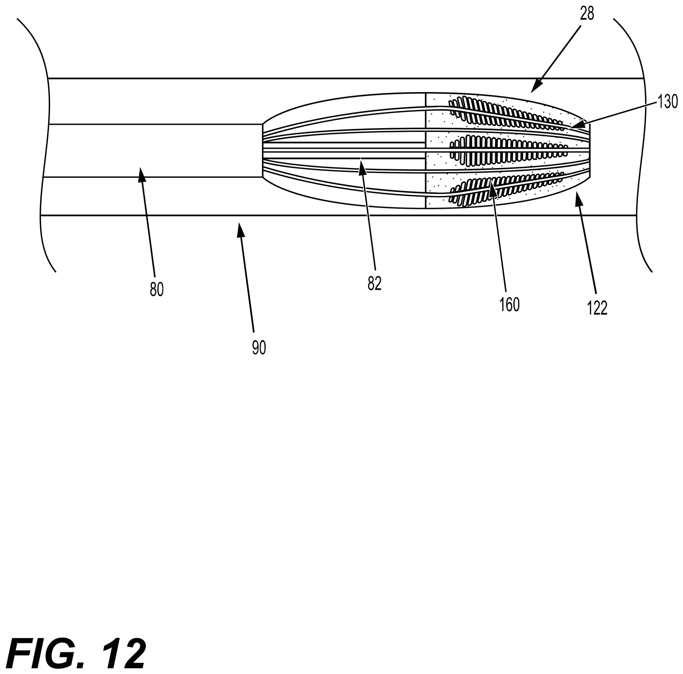

The framework 120 can include a flexible, resilient material (e.g., a shape-memory alloy such as nickel-titanium, also known as Nitinol or stainless steel) that is shape set to be biased to bend outwardly to an expanded configuration, as shown in FIGs. 2 and 3. The spines 122 can be curved or extend approximately linearly in the outward direction to respective distal ends 122-1. In the expanded configuration, the framework 120 resembles ribs of an umbrella. Due to the flexible, resilient nature of the material, the spines 122 can also be moved to a collapsed configuration (an example of the collapsed configuration can be seen in FIG. 12) where the spines 122 extend approximately along the longitudinal axis 60.

The end effector further includes a flexible membrane 130 connected to the framework 120 and extending to a distal end 130-1. Specifically, the membrane 130 is connected to each spine 122 such that the distal end 130-1 surrounds/encompasses the longitudinal axis 60, with the spines 122 biasing the membrane 130 to the expanded configuration and also being movable to the collapsed configuration. As seen in FIGs. 2-3, the distal end 130-1 of the membrane extends beyond the distal ends 122-1 of the spines 122. This membrane 130 serves to enhance the atraumaticity of the tip of the end effector 100 and to protect the subject from sharp edges. The membrane 130 can include a flexible biocompatible material, such as a polymer. Further uses, advantages, and materials of the membrane 130 are detailed later in this document. It is noted that the flexible membrane 130 in FIGs. 4A-7 and 10 is depicted without cross-sectional hatching to more easily differentiate between components shown in the respective figures.

The membrane 130 can include one or more sheets/layers fused together proximate the framework 120 into a single, contiguous, generally planar insulative mass 130. For example, the membrane 130 can include a first layer 132 that forms a distal-facing exterior surface of the end effector 100, a second layer 134 that forms a proximal-facing exterior surface of the end effector 100, and a third layer 136 disposed between the first layer 132 and the second layer 134. As seen best in FIG. 3, the second layer 134, due to the overall shape of the end effector 100 in the expanded configuration, defines a volume V with an open end (i.e., the proximal side of the volume V).

In some examples, one or more of the layers of the membrane 130 (e.g., third layer 136) comprises a dielectric material. For examples, it can include high dielectric sheets/tiles, or it can be formed using high dielectric TPU doping. The various configurations shown in the figures provide differing levels of electrical insulation between electrodes on opposite sides of the membrane 130. The electrical insulation may be tailored to direct electric field lines, and thereby electroporation of cells within target tissue, between bipolar pairs of ablation electrodes on opposite sides of the membrane 130 during PFA application.

The membrane 130 can be heat formed around at least a portion of a first flexible circuit 110 (FIG. 4A, discussed in greater detail below), a second flexible circuit 150 (FIG. 4A, discussed in greater detail below), and the framework 120, making the end effector 100 easy to manufacture compared with other labor-intensive processes. The polymer can include TPU, silicone, or other heat formed or shaped material which lends itself to said heat forming. In the expanded configuration, the membrane 130, by virtue of its connection to the spines 122, forms a generally semi-spherical or semi-conical shape, similar to that of the canopy of an umbrella. It is additionally noted that, depending on the tension of the membrane 130 wrapped around the spines 122, the shape may resemble a polygonal pyramid (e.g., an octagonal pyramid in the case there are 8 spines 122). Collapsing the umbrella shape from the expanded configuration can be achieved by virtue of the engagement between the spines 122 and the delivery sheath when the end effector 100 is pulled in the proximal direction. In some examples, the spines 122 can invert such that the distal ends 122-1 thereof point in the distal direction when collapsed. In other examples, an actuator can be connected to the framework to move the spines 122 in unison to the collapsed or expanded configuration. The umbrella shape, with its solid/continuous membrane form, aids in keeping the electrodes 160 at a known distance apart from one another, which is important to lesion formation and selection of the ablation parameters.

Making reference to FIGs. 2A and 34A, the end effector 100 further includes a first flexible circuit 110 and a second flexible circuit 150 that extends radially around the longitudinal axis 60, e.g., in a sun ray pattern. Each flexible circuit 110, 150 includes a flexible substrate 111, 151 (i.e., a first flexible substrate layer 111 and a second flexible substrate layer 151) that can be a continuous flexible circuit member that extends entirely, or substantially entirely, around the longitudinal axis 60, or each flexible circuit 110, 150/flexible substrate 111, 151 can be subdivided into multiple flexible circuit sections that conjunctively extend around the longitudinal axis 60 and are circumferentially spaced from each other. The first flexible circuit 110 and second flexible circuit 150 each include a plurality of electrodes 160 electrically connected to the PIU 30 via electrical interconnections (e.g., traces). In instances where the flexible circuits 110, 150 are subdivided into multiple sections, these sections are each positioned near a spine 122, and each respective flexible substrate 111, 151 can include electrodes 160 thereon. While not explicitly illustrated, it is noted that the end effector 100 can also include other layers, such as a location sensing loop layer for sensing a position or shape of the end effector 100.

Each respective flexible substrate layer 111, 151 comprises a bio-compatible material. In some examples, each flexible substrate layer 111, 151 is formed entirely from or about entirely from the bio-compatible material. In some examples, the flexible substrate layer is formed from polyimide, copper, LCP, nitinol substrate, thermoplastic polyurethane (TPU), silicone, thermoset resin, or other polymeric substrates. In some examples, each flexible substrate layer 111, 151 described herein can be made primarily of polyimide. In other examples, it can be made of any of biocompatible polyimides, glass-reinforced epoxy laminate materials, copper, or graphene, alone or in combination.

As discussed above, the flexible circuits 110, 150 are disposed on the membrane 130 that extends around the longitudinal axis 60. More specifically, the first flexible circuit 110 is disposed on a first surface 136A (that faces away from the longitudinal axis 60) of the membrane 130 and the second flexible circuit 151 is disposed on a second surface 136B (that faces towards from the longitudinal axis 60) of the membrane 130 that is opposite the first surface 136A (relative to the spines 122).

Moreover, the membrane 130 can be contiguous to the contact surfaces of the electrodes 160 so that only the contact surfaces of at least a portion of the plurality of electrodes 160 are exposed to the ambient environment. The contact surfaces of the electrodes 160 can be flush with an outer surface of the membrane 130, recessed relative to the outer surface of the membrane 130, or protrude therefrom. As used herein, "ambient environment" refers to the external environment such as the organ in which the end effector 100 is deployed or in the operating theater prior to being deployed in the biological organ. The membrane 130 at least partially encapsulates or spaces the different layers of the end effector 100 (e.g., the flexible circuits 110, 150 (including the substrate layers 111, 151) and the framework 120) along a vertical axis 62. See, for example, FIG. 4A, which shows the flexible circuits 110, 150 spaced from the spine 122 via the third layer 136 of the membrane 130, which is disposed between the flexible circuits 110, 150 and the spines 122.

It is noted that not all of the electrodes 160 on the flexible circuits 110, 150 described herein need be exposed through the insulative material 130 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood.

With continued reference to FIGs. 2A, 3-4A, the electrodes 160 comprise plural ablation (or treatment) electrode sets 161, each ablation electrode set 161 being positioned proximal a respective spine 122. These associated spines 122 and electrodes 160 are referred to as electrode/spine regions (or electrode and spine regions). As seen best in FIG. 4A, in some examples, not every spine 122 has an associated electrode set. The example of FIGs. 2A and 3-4A presents an end effector 100 where an alternating pattern of ablation electrodes 161 adjacent the spine 122 and no ablation electrodes 161 adjacent the spine 122 is employed. Put another way, in that example, there are more spines 122 than there are sets of ablation electrodes 161 (e.g., double the number of spines 122 compared with sets of electrodes 161). In the example of FIG. 2A, there are four electrode/spine regions 102A-102D (i.e., a first electrode/spine region 102A, a second electrode/spine region 102B, a third electrode/spine region 102C, and a fourth electrode/spine region 102D).

FIGs. 2B and 4B depicts a variant views, similar to that of FIGs. 2A and 4A respectively, where each spine 122 has an associated electrode set. Put another way, in the example of FIGs. 2B and 4B, there are an equal number of spines 122 and sets of ablation electrodes 161. In the example of FIG. 2B, there are eight electrode/spine regions 102A-102H (i.e., a first electrode/spine region 102A, a second electrode/spine region 102B, a third electrode/spine region 102C, a fourth electrode/spine region 102D, a fifth electrode/spine region 102E, a sixth electrode/spine region 102F, a seventh electrode/spine region 102G, and an eighth electrode/spine region 102H), but is otherwise structurally configured the same as the example of FIGs. 2A and 3-4A. Therefore, it will be appreciated by those skilled in the art that description regarding the electrode configuration of, e.g., FIG. 4A also applies to the electrode configuration of FIG. 4B, unless specifically noted to the contrary.

Each electrode of the respective ablation electrode set 161 is vertically spaced (along the vertical axis 62) from its associated/adjacent spine 122. As can be seen in FIGs. 2A and 3-4A, each ablation electrode 161 is provided on a lateral side of its associated/adjacent spine 122 of its electrode/spine region and extends along a predetermined length thereof.

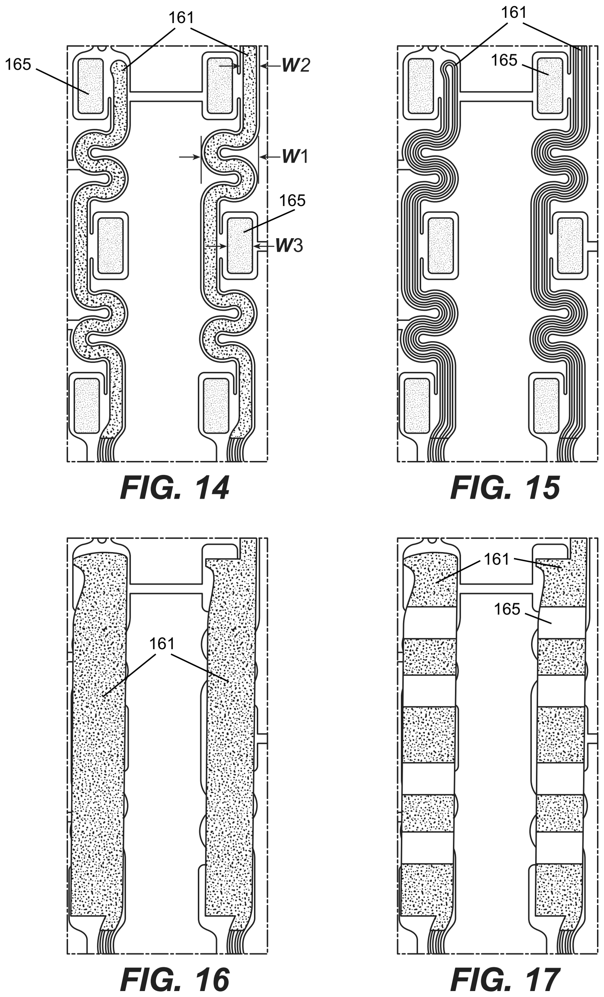

The left side of FIG. 4A is example electrode configuration on both sides (upper and lower) of the example end effector 100 including elongated ablation electrodes 161, each ablation electrode defining a treatment region. While not shown in this figure, the diagnostic electrodes 165 can be positioned in the two ablation electrode regions (see, e.g., FIG. 14 discussed below) or outside the treatment regions, tissue contact electrodes (discussed in greater detail below) positioned between the two upper ablation electrode regions, and a reference electrode (discussed in greater detail below) positioned in a region of the end effector 100 that does not contact tissue.

An axis of each spine 122 can serve as a line of symmetry for its respective associated electrodes 160 (i.e., the electrodes 160 that are disposed proximal thereto). As illustrated, the electrode configuration is symmetric about the spine 122. Preferably, at least the ablation electrodes 161 are symmetric to each other with respect to the spine 122. In an alternative example, the electrode configuration may not be symmetric about the line of symmetry.

As seen in FIG. 4A, in some examples, each ablation electrode set 161 can include four electrodes 161A-161D (also referred to herein as treatment or ablation electrodes), including a first ablation electrode 161A, a second ablation electrode 161B, a third ablation electrode 161C, and a fourth ablation electrode 161D. A first pair of ablation electrodes 161A, 161B is disposed on the first flexible circuit 110 proximal to one another and their associated spine 122. Similarly, a second pair of ablation electrodes 161C, 161D is disposed on the second flexible circuit 150 proximal to one another and their associated spine 122, with the membrane 130 between them such that a mirror image configuration is defined. The first pair of ablation electrodes 161 is configured to be positioned against tissue during treatment. When the upper side of the end effector 100 is in contact with tissue, corresponding electrodes 161C, 161D on the opposite side are in contact with body fluid (e.g., blood) and can act as respective reference electrodes for those electrodes 161A, 161B in contact with tissue.

The illustrated section of the membrane 130 in FIG. 4A, proximal the spine 122, has a left half which is left of the line of symmetry and a right half which is right of the line of symmetry. The first ablation electrode 161A defines a first electrode region, which is entirely on the left side of its associate spine 122. The second ablation electrode 161B defines a second electrode region, which is entirely in the right half of its associated spine 122. The third and fourth ablation electrodes 161C, 161D similarly define third and fourth electrode regions which are respectively entirely on the left and right sides of the spine 122.

The ablation electrodes 161A-161B can take a number of shapes/designs, such as a serpentine shape, provided as a strip, provided as a bar, or provided as an articulating bar. FIGs. 14-17 depict exemplary configurations thereof, which are described in greater detail below. In general, ablation electrodes 161C, 161D are illustrated on the second (lower) side opposite the ablation electrodes 161A, 161B on the first (upper) side. The opposite ablation electrodes 161C, 161D overlap the ablation electrodes 161A, 161B on the first side preferably overlapping a majority of the ablation electrodes 161A, 161B on the first side 101a, and may be symmetric to the ablation electrodes 161C, 161D on the first side with respect to a plane defined by the membrane 130 when laid flat (e.g., as depicted in FIG. 4A for illustrative purposes). The cross-section of the end effector 100 is simplified to omit certain features, such as, but not limited to, electrical traces, for the sake of illustration. The ablation electrodes 161A-161D are illustrated sunken into the membrane 130, but may alternatively protrude therefrom on each respective side. The ablation electrodes 161A-161D can have approximately equal surface area to each other.

In some examples, the ablation electrodes 161A-161D have a length that is at least half of a total radius of the end effector 100 as measured from the distal end 130-1 of the flexible membrane 130 to the attachment section 121. In some examples, the ablation electrodes 161A-161D extend to approximately the distal end 130-1 of the flexible membrane 130-1.

The ablation electrodes 161A-161D of each set are connected to the ablation energy generator 50. With the configuration detailed herein, a plurality of ablation configurations can be employed. In some examples, and as mentioned above the ablation electrodes 161C, 161D on the second side can function as respective reference electrodes for the respective opposite electrode 161A, 161B on the first side of the membrane 130 when the first side is in contact with tissue. Additionally, or alternatively, the ablation electrodes 161 on opposite sides can be paired to provide bipolar PFA electrical signals between the paired ablation electrodes 161. Additionally, or alternatively, electrodes 161 on the same side can be paired to provide bipolar PFA electrical signals between the paired electrodes. The bipolar PFA electrical signals may be monophasic or biphasic. It is noted that while each pair of first electrodes (161A, 161B) are described as discrete electrodes, it is within the scope of the claimed technology that both electrodes in each of the first pair (e.g., 161A, 161B) or the second pair (e.g., 161C, 161D) are electrically connected to form a single ablation electrode. Specifically, the first pair of electrodes 161A, 161B can be electrically connected together, via the spine 122 between the electrodes or directly at the generator 50, to define a single first electrode of the plurality of first electrodes disposed on the first surface of the membrane. Similarly, the second pair of electrodes 161C and 161D can be electrically connected together, via the spine 122 between them or directly at the generator 50, to define a single second electrode of the plurality of second electrodes disposed on the second surface of the membrane. It is further noted that each of the electrode 161A, 161B, 161C, 161D can be configured as a diagnostic electrode (which receives electrical signals from the tissues instead of transmitting electrical signals from the generator for ablation).

The illustrated end effector 100 includes optional diagnostic electrodes 165 on the first side of the membrane 130 that are electrically isolated from the ablation electrodes 161. The diagnostic electrodes 165 are configured to receive electrical signals from tissue to map cardiac tissue and detect arrhythmia. As illustrated, the diagnostic electrodes 165 are arranged as a pair with a first diagnostic electrode 165 on the left side of each spine 122 and a second diagnostic electrode 165 on a right side of each spine 122.

The end effector 100 can include optional tissue contact quality electrodes positioned in closely spaced pairs such that impedance measured across the respective tissue contact quality electrode pair indicates that electrodes of that pair are both in contact with tissue.

The end effector 100 includes an optional reference electrode (it is noted that, while primarily described as a diagnostic electrode, reference number 165 can also be considered to denote an exemplary reference electrode or any other electrode discussed herein) disposed on the first side of the membrane 130. Additionally, or alternatively, the end effector 100 can include a reference electrode similarly disposed on the second side membrane. The reference electrode(s) may be used for ECG gathering in either, unipolar, bipolar (split or close pair) or may be a reference. The number can be as few as four per side but as many as needed (and can fit with trace limitations). The signals from the reference electrode(s) may also be used for contact information to determine what portion of the paddle has contact or proximity to the tissue. The end effector 100 includes one or more sections which lack any ablation electrode. The reference electrode(s) may be disposed in this section. As discussed herein, an ablation electrode, diagnostic electrode, or tissue contact electrode may be used as a reference electrode for a corresponding electrode on the opposite side in contact with tissue. Each of these electrodes are positioned in the distal portion of the end effector 100 so that they may be positioned in contact with tissue if so desired by the physician 24. Preferably, the reference electrode is positioned such that the physician 24 is unable, or at least very unlikely, to position the reference electrode in contact with tissue but nevertheless in relatively close proximity to electrodes which are configured to contact tissue.

In some examples, at least the ablation electrodes, diagnostic electrodes, and tissue contact electrodes are flush with the outer surfaces of the membrane 130 to provide a first flush surface to the end effector 100 corresponding to the first side of the membrane 130 and a second flush surface to the end effector 100 corresponding to the second side of the membrane 130. The ablation electrodes 161 and any combination of other electrodes 165 (as well as the others discussed herein) may include an exposed conductive layer in a flexible printed circuit board, may include silver epoxy, or conductive ink.

Further details on exemplary configurations of the electrodes can be found with respect to the description of FIGs. 14-17.

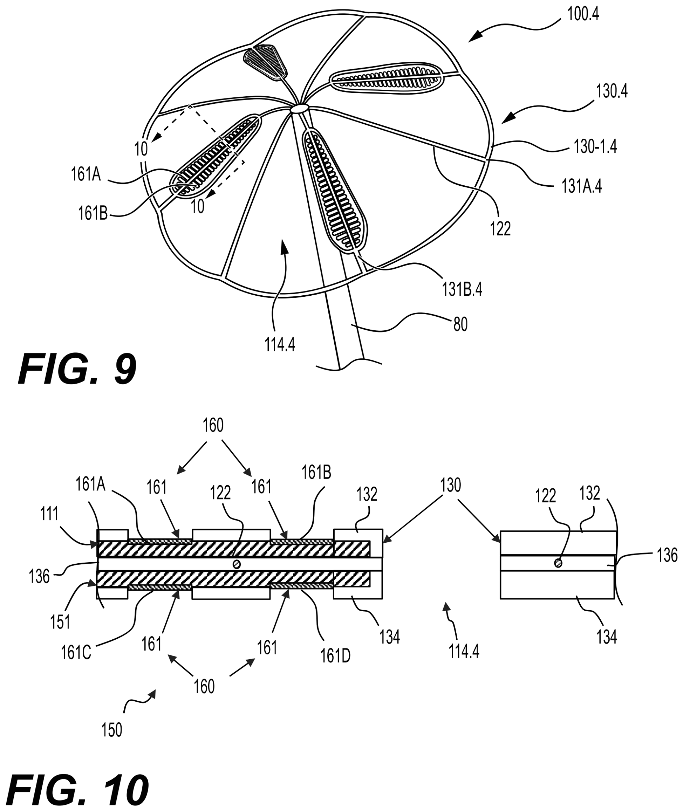

As mentioned above, the membrane 130 can include a third layer 136 between the first/upper and second/lower sides of the end effector 100. The third layer can include a polymeric body region extending a width of the respective region associated with each ablation electrode set 161 and spine 122. In some examples, such as ones where the flexible circuits 110, 150 are embodied as discrete, discontinuous circuits, the third layer 136 can be provided as respective thinned polymer fill-in regions that connect adjacent flexible circuits 110, 150 and which lack a framework or any electrical circuitry. Configured as such, the membrane 130 may provide additional electrical insulation between electrodes on opposite sides of the end effector 100 (compared to examples that include openings, such as seen in FIGs. 9-10) while maintaining sufficient flexibility for manipulation to position against tissue and transfer through a sheath. As mentioned above, this third layer 136 can also be embodied as a high dielectric layer between each side and extending around the end effector 100. The dielectric layer 134 is overlapping and parallel to each of the ablation electrodes 161A-161D. The high dielectric layer can include a ceramic doped polymer to provide additional electrical insulation between electrodes on opposite sides of the membrane 130 compared to polymer alone while still providing sufficient flexibility. Some portions of the end effector 100 can the high dielectric layer 134, lacking a framework, and lacking electrical circuitry. In some examples, the third layer 134 can be embodied as high dielectric tiles between each side and configured to overlap to collapse with the end effector 100 into a delivery sheath. The high dielectric tiles can include ceramic plates that extend longitudinally through each segment of the body and overlap between segments. The high dielectric tiles or longitudinally extending ceramic plates may be angled with respect to a plane defined by the membrane 130 when laid flat such that the tiles/plates are configured to overlap, longitudinal side on longitudinal side upon retraction of the end effector into a sheath.

FIGs. 5A-5C depict exemplary ablation configurations for application of bipolar PFA electrical pulses in examples where the end effector includes four ablation electrodes 161A-161D. In these figures, the first and second ablation electrodes 161A, 161B face outwardly towards tissue while the third and fourth ablation electrodes 161C, 161D (which are disposed in the volume V) face away from the tissue. In use, the first and second ablation electrodes 161A, 161B can contact the tissue (or be disposed immediately adjacent thereto), and the ablation energy generator 50 selectively activates pairs of the ablation electrodes to apply pulse field ablation pulses. In some examples, the pulses have a voltage of approximately 600 volts (V) to about 1,200 V. In other examples, the pulses have a voltage from 600 V to 2,600 V. In some examples, a bipolar PFA electrical signal includes 60 pulses with a magnitude of approximately 1,200 V and a total duration of approximately 4 seconds. In some examples, the bipolar pulses may include an interpulse delay of approximately 2 microseconds. The pattern of ablation electrode pairings can be adapted based on the total number of ablation electrodes in the end effector 100 as discussed in greater detail as understood by a person skilled in the pertinent art informed by the disclosure herein.

FIG. 5A depicts one such exemplary ablation configuration. In this example, the ablation electrodes 161A-161B are activated diagonally/cross-body and sequentially. The generator 50 can alternatingly energize a first pair of the ablation electrodes 161 to apply pulses between the first ablation electrode 161A(+) and fourth ablation electrode 161D(-), followed by energizing a second pair of the ablation electrodes 161 to apply a pulse between the second ablation electrode 161B(+) and the third ablation electrode 161C(-), such that only one pair of ablation electrodes 161 of each set is activated at a time.

In this example, the ablation electrodes 161 in a pair are symmetric with respect to the spine 122 and are on opposite sides of the membrane 130. A voltage is applied across electrodes in pairs of electrode regions in which, for each pair, ablation electrodes in a first electrode region is in contact with tissue and the other electrode region is on an opposite side of the end effector and across the spine 122 from the first electrode region. FIG. 5A illustrates ablation electrode 161A having a positive charge while the paired ablation electrode 161D has a negative charge. This illustrates a positive voltage pulse configured to induce electroporation in tissue in contact with the upper side of the membrane 130. A biphasic pulse may be applied in which the polarity switches or alternates between as shown in FIG. 5A to one in which positive charge is on the ablation electrode 161D not in contact with tissue while negative charge is on the ablation electrode 161A in contact with tissue. A train of bipolar pulses (which may include biphasic or monophasic pulses) can be applied between the bipolar ablation electrode pair 161A, 161D as shown in FIG. 5A, then subsequently, a train of bipolar pulses can be applied between the other bipolar ablation electrode pair 161B, 161C in the same manner as described above.

Put another way, FIG. 5A depicts an end effector 100 that is configured to provide electrical pulses 600-1,200 V in amplitude between bipolar pairs which include an ablation electrode (e.g., first ablation electrode 161A) on the upper side of the membrane 130 and in contact with tissue, and an ablation electrode (e.g., fourth ablation electrode 161D) on the lower side of the membrane 130 and not in contact with tissue. Similarly, ablation electrodes 161B, 161C can be paired.

In some examples, cardiac electrical signals may be measured from one or more diagnostic electrode(s) disposed on the side of the membrane 130 in contact with tissue. In some embodiments, tissue contact may be measured from a pair of tissue contact electrodes disposed on the side of the membrane 130 in contact with tissue. In some examples, a reference electrical signal may be measured from a reference electrode disposed on the side of the membrane 130 in contact with tissue, wherein the reference electrode itself is not in contact with tissue.

FIG. 5B depicts another exemplary ablation configuration. In this example, rather than applying the pulses diagonally, the pulses are applied vertically (relative to the orientation of FIG. 5B) such that the ablation electrodes in each pair are overlapping and opposite sides of the membrane 130. The generator 50 can simultaneously or alternatingly energize a first pair of the ablation electrodes 161 to apply pulses between the first ablation electrode 161A(+) and third ablation electrode 161C(-), as well as energize a second pair of the ablation electrodes 161 to apply a pulse between the second ablation electrode 161B(+) and the fourth ablation electrode 161D(-). A voltage is applied across ablation electrodes 161A, 161B in contact with tissue to ablation electrodes 161C, 161D on an opposite side of the membrane 130 and not in contact with tissue. A biphasic pulse may be applied in which the polarity switches or alternates between as shown in FIG. 5B to one in which positive charge is on ablation electrodes 161C, 161D not in contact with tissue while negative charge is on ablation electrodes 161A, 161B in contact with tissue. A train of bipolar pulses can be applied between the bipolar ablation electrode pairs. As illustrated, both ablation electrodes 161A, 161B in contact with tissue can be activated simultaneously. Alternatively, a train of bipolar pulses may be applied to the first pair (161A, 161C) of ablation electrodes while the second pair (161B, 161D) is deenergized, and subsequently a train of bipolar pulses may be applied to the second pair of ablation electrodes while the first pair is deenergized.

FIG. 5C depicts yet another exemplary ablation configuration. In this example, rather than applying the pulses diagonally or vertically, the pulses are applied horizontally (relative to the orientation of FIG. 5B) across the upper pair of electrodes. In other words, the ablation electrodes 161 in each pair are symmetric with respect to the spine 122 and on the same side of the membrane 130. Specifically, the generator 50 can energize the upper pair of the ablation electrodes 161 to apply pulses between the first ablation electrode 161A(+) and second ablation electrode 161B(-), while the third and fourth ablation electrodes 161C, 161D are not activated. As illustrated, a voltage is applied across ablation electrodes 161A, 161B on the side membrane 130 in contact with tissue. The ablation electrodes 161C, 161D not in contact with tissue can function as reference electrodes. A biphasic pulse may be applied in which the polarity switches or alternates between as shown in FIG. 5C to one in which positive charge is on the right ablation electrode 161B while negative charge is on the left ablation electrode 161A. A train of bipolar pulses can be applied between the bipolar ablation electrode pair 161A, 161B.

It is noted that cross-region ablative pulses with similar patterns to those described with respect to FIGs. 5A and 5C can be employed without departing from the spirit and scope of the present disclosure. By way of example, and with reference to the example of FIG. 2A with four electrode/spine regions 102A-102D, a train of bipolar pulses (which, as mentioned above, may include biphasic or monophasic pulses) can be applied between one or more ablation electrodes 161 in the first electrode/spine region 102A and one or more ablation electrodes 161 in the second electrode/spine region 102B, followed by a train of bipolar pulses between one or more ablation electrodes 161 in the second electrode/spine region 102B and one or more ablation electrodes 161 in the third electrode/spine region 102C, followed by a train of bipolar pulses between one or more ablation electrodes 161 in the third electrode/spine region 102C and one or more ablation electrodes 161 in the fourth electrode/spine region 102D, followed by a train of bipolar pulses between one or more ablation electrodes 161 in the fourth electrode/spine region 102G and one or more ablation electrodes 161 in the first electrode/spine region 102H. By way of further example, and with reference to the example of FIG. 2B with eight electrode/spine regions 102A-102H, a train of bipolar pulses (which, as mentioned above, may include biphasic or monophasic pulses) can be applied between one or more ablation electrodes 161 in the first electrode/spine region 102A and one or more ablation electrodes 161 in the second electrode/spine region 102B, followed by a train of bipolar pulses between one or more ablation electrodes 161 in the third electrode/spine region 102C and one or more ablation electrodes 161 in the fourth electrode/spine region 102D, followed by a train of bipolar pulses between one or more ablation electrodes 161 in the fifth electrode/spine region 102E and one or more ablation electrodes 161 in the sixth electrode/spine region 102F, followed by a train of bipolar pulses between one or more ablation electrodes 161 in the seventh electrode/spine region 102G and one or more ablation electrodes 161 in the eighth electrode/spine region 102H.

Of course, other cross-region ablative patterns can be used. For another example, and with continued reference to FIG. 2B, a train of bipolar pulses (which may include biphasic or monophasic pulses) can be applied between one or more ablation electrodes 161 in the first electrode/spine region 102A and one or more ablation electrodes 161 in the fifth electrode/spine region 102E, followed by a train of bipolar pulses between one or more ablation electrodes 161 in the second electrode/spine region 102B and one or more ablation electrodes 161 in the sixth electrode/spine region 102F, followed by a train of bipolar pulses between one or more ablation electrodes 161 in the third electrode/spine region 102C and one or more ablation electrodes 161 in the seventh electrode/spine region 102G, followed by a train of bipolar pulses between one or more ablation electrodes 161 in the fourth electrode/spine region 102D and one or more ablation electrodes 161 in the eighth electrode/spine region 102H. Similar techniques can be employed for the example of FIG. 2A.

In even further examples, cross-region ablative pulses can have even further bipole patterns that employ two or more electrode/spine regions as the positive electrode in the bipole. By way of example, and with reference to the example of FIG. 2A, a train of bipolar pulses can be applied between ablation electrodes 161 in the first and second electrode/spine regions 102A, 102B (functioning as the positive electrode in the bipole) and one or more ablation electrodes 161 in the third electrode/spine region 102C (functioning as the negative electrode in the bipole), followed by a train of bipolar pulses applied between ablation electrodes 161 in the second and third electrode/spine regions 102B, 102C (functioning as the positive electrode in the bipole) and one or more ablation electrodes 161 in the fourth electrode/spine region 102D (functioning as the negative electrode in the bipole), followed by a train of bipolar pulses applied between ablation electrodes 161 in the third and fourth electrode/spine regions 102C, 102D (functioning as the positive electrode in the bipole) and one or more ablation electrodes 161 in the first electrode/spine region 102A (functioning as the negative electrode in the bipole), followed by a train of bipolar pulses applied between ablation electrodes 161 in the fourth and first electrode/spine regions 102D, 102A (functioning as the positive electrode in the bipole) and one or more ablation electrodes 161 in the second electrode/spine region 102B (functioning as the negative electrode in the bipole).

By way of example, and with reference to the example of FIG. 2B, a train of bipolar pulses can be applied between ablation electrodes 161 in the first and second electrode/spine regions 102A, 102B (functioning as the positive electrode in the bipole) and ablation electrodes 161 in the fifth and sixth electrode/spine regions 102E, 102F (functioning as the negative electrode in the bipole), followed by a train of bipolar pulses applied between ablation electrodes 161 in the second and third electrode/spine regions 102B, 102C (functioning as the positive electrode in the bipole) and ablation electrodes 161 in the sixth and seventh electrode/spine regions 102F, 102G (functioning as the negative electrode in the bipole), followed by a train of bipolar pulses applied between ablation electrodes 161 in the third and fourth electrode/spine regions 102C, 102D (functioning as the positive electrode in the bipole) and ablation electrodes 161 in the seventh and eighth electrode/spine regions 102G, 102H (functioning as the negative electrode in the bipole), followed by a train of bipolar pulses applied between ablation electrodes 161 in the fourth and fifth electrode/spine regions 102D, 102E (functioning as the positive electrode in the bipole) and ablation electrodes 161 in the eighth and first electrode/spine regions 102G, 102A (functioning as the negative electrode in the bipole).

The above ablation configuration examples are non-limiting and are merely intended to elucidate certain ways in which the presently described technology can be implemented. In all of the above-described examples, the goal of the configuration of the ablation bipoles and sequencing is to achieve a circumferential lesion without needing to reposition the end effector 100.

Having described various exemplary ablation patterns that can be employed, the present disclosure now turns to exemplary alternative physical forms the electrodes 160 or the overall end effector 100 can take. Unless explicitly noted to the contrary, it will be appreciated that the previously described ablation configurations can be applied to any of the following exemplary end effectors as well.

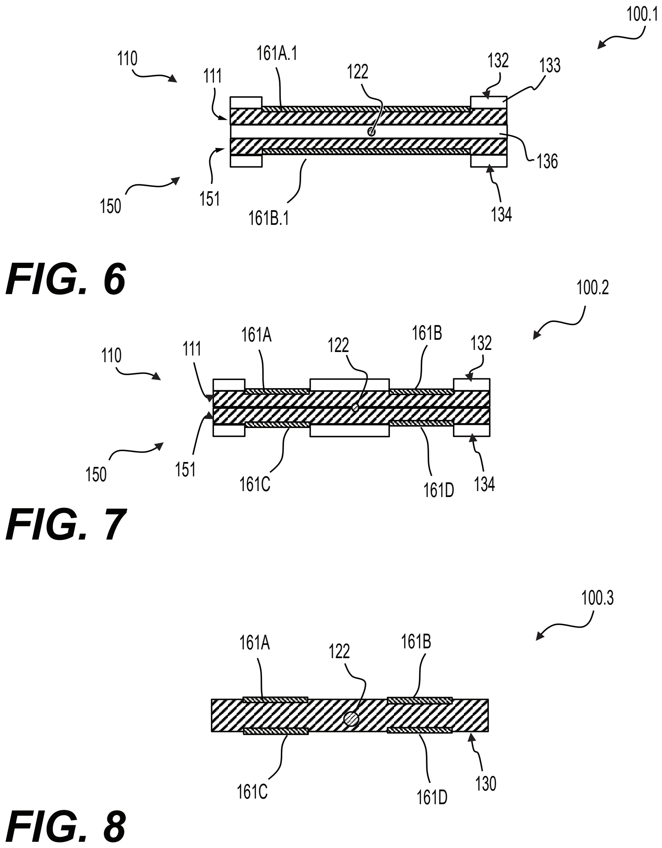

FIG. 6 depicts a variant 100.1 of the above-described end effector 100. This example is identical to the previously described one with the exception that, rather than providing a set of four ablation electrodes 161 proximal the spines 122, a single pair of ablation electrodes is provided. Specifically, a first ablation electrode 161A.1 is disposed on the first flexible substrate 111 and second ablation electrode 161B.1 is disposed on the second flexible substrate 151 in a mirror configuration. This example can employ the ablation configuration discussed with respect to FIG. 5B or the exemplary cross-region ablative patterns described in the preceding paragraphs. It is additionally noted that, in some examples, a single ablation electrode 161A.1 can be provided on the upper side in each electrode/spine region 102A-102H, with the above-described cross-region ablative pulses being employed to achieve IRE.

FIG. 7 depicts yet another variant 100.2 of the above-described end effector 100. This variant 100.2 can be configured identically to that of the end effector 100, with the third layer 136 of the membrane omitted such that the two flexible substrates 111, 151 can be provided directly on the spines 122 or in contact with one another.

FIG. 8 depicts yet another variant 100.3 of the above-described end effector 100. In this example, the membrane 130 functions as the flexible circuit, with the first and second ablation electrodes 161A, 161B and second and third ablation electrodes 161C, 161D being provided on opposing surfaces thereof.

Reference is now made to FIGs. 9-10, which depict yet another variant 100.4 of the above-described end effector 100. In this example, the membrane 130.4 is configured with voids 114.4 defined therethrough, with each void 114.4 having a closed perimeter bounded by the membrane 130.4. The distal end 130-1.4 of the membrane 130.4 forms an annular bridge 131A.4 with the spines 122 and electrodes 160 connected to the annular bridge 131A.4 via connecting sections 131B.4. The voids 114.4 allow for easier collapsing for passage through the sheath and also allow for more blood flow. The volume of the removed material (compared to the solid umbrella form) is omitted and thus there is less material to fit in a given collapsed cross section. The voids 114.4 can be sized, shaped, or otherwise configured to allow the end effector 100 to flex through a sheath catheter. The voids 114.4 can be sized, shaped, or otherwise configured to collapse or expand when the end effector 100 is pressed against a non-planar tissue surface to provide conformal contact to the non-planar tissue surface. It is recognized that consistent contact of ablation electrodes 161 to the non-planar tissue may produce improved lesions compared to less conformal electrode contact. The membrane distal end 130-1.4 helps to maintain the relative positioning between the electrodes 160. The annular bridge 131A.4 (or additional bridges) can also be located at varying distances from the longitudinal axis 60 along the membrane 130.4.

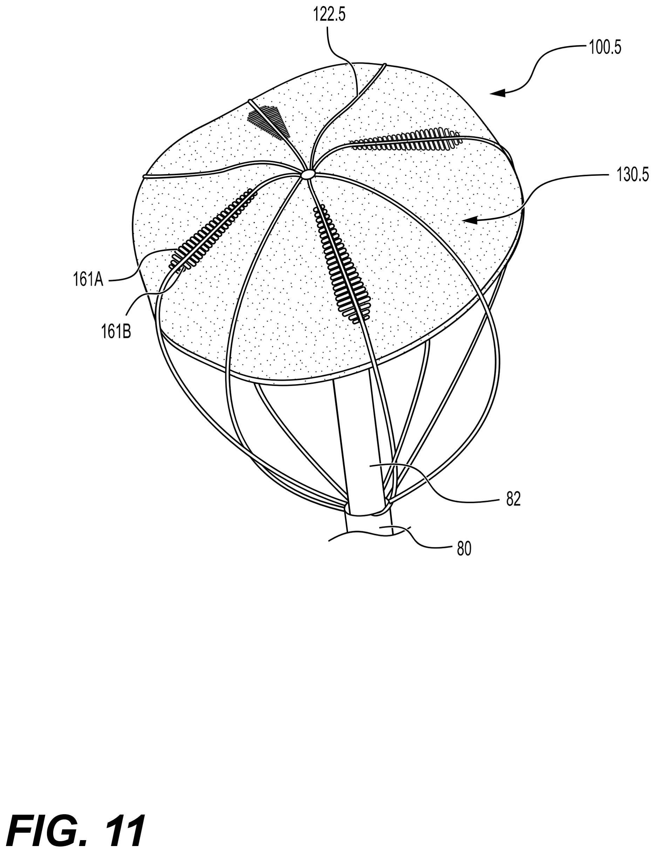

Reference is now made to FIGs. 11-12, which depict yet another variant 100.5 of the above-described end effector 100. In this example, the spines 122.4 are configured in the form of a basket catheter, where first ends of the spines 122.4 are connected to a first probe shaft 80, and second ends of the spines 122.4 are connected to a second probe shaft 82 (e.g., a pusher tube or pusher wire) which can be used to expand and collapse the basket (see the collapsed configuration in FIG. 12, where the end effector 100.5 is disposed in a sheath 90).

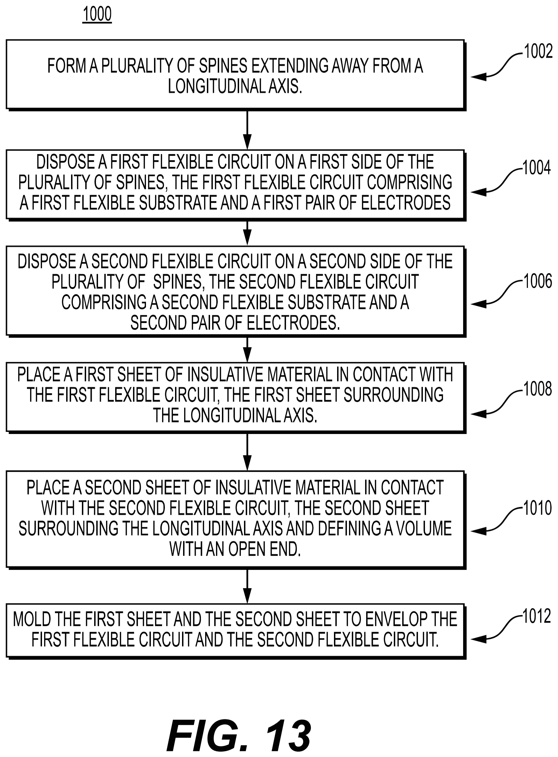

FIG. 13 is a flow chart depicting a method 1000 of making an end effector 100 in accordance with the present disclosure. The method 1000 includes forming 1002 a plurality of spines extending away from a longitudinal axis. The method 1000 includes disposing 1004 a first flexible circuit on a first side of the plurality of spines, the first flexible circuit comprising a first flexible substrate and a first ablation electrode. The method 1000 includes disposing 1006 a second flexible circuit on a second side of the plurality of spines, the second flexible circuit comprising a second flexible substrate and a second ablation electrode. The method 1000 includes placing 1008 a first sheet of insulative material in contact with the first flexible circuit, the first sheet surrounding the longitudinal axis. The method 1000 includes placing 1010 a second sheet of insulative material in contact with the second flexible circuit, the second sheet surrounding the longitudinal axis and defining a volume with an open end. The method includes molding 1012 the first sheet and the second sheet to envelop the first flexible circuit and the second flexible circuit such that the first pair of ablation electrodes, the second pair of ablation electrodes, and one of the spines are disposed proximal to one another.

In some examples, prior to molding 1012 the first sheet and the second sheet, the method 1000 further includes placing a third sheet of insulative material between the first flexible circuit and the second flexible circuit and in contact with the spine, and molding the third sheet such that the first flexible circuit and the second flexible circuit is spaced apart from the plurality of spines.

Turning now to FIGs. 14-17, alternative/exemplary electrode configurations are depicted therein. In some examples, the electrodes can have a fishbone configuration. Examples of electrodes with fishbone configurations are disclosed in U.S. Patent No. 11,642,165B2, which is incorporated herein by reference in its entirety. Other advantageous designs are detailed below. In general, each ablation electrode can be designed with shape based on trade-off between total edge area, total contact area, and open space for non-ablation electrodes. It is recognized that a larger ratio of surface area to perimeter may effectively deliver the ablation energy while reducing the chance of electrical arcing. It is also recognized that it is desirable to avoid arcing. Conversely, serpentine electrodes increase mechanical flexibility of the paddle. It is recognized that flexibility of the electrode may result in improved tissue contact and catheter longevity, but the geometry of the serpentine electrode has a lower area to perimeter ratio compared to a solid electrode.

FIG. 14 is an illustration of a first electrode configuration for the example end effector 100 including one example configuration of a treatment/ablation electrode 161 and diagnostic electrodes 165. In the illustrated example, each ablation electrode 161 is divided into two longitudinally elongated segments that extend along their associated spine 122. In this example, longitudinally elongated segments on a left side of the spine 122 form a first ablation electrode 161, and longitudinally elongated segments on a right side of the spine 122 form a second ablation electrode 161. By way of example, with this configuration, the example of FIG. 4A would have eight elongated segments in total (per each associated spine 122), with four on the upper side and four on the lower side of the membrane 130. It will be appreciated, of course, that in some examples, the aforementioned four electrodes 161A-161D each have a single elongated segment such that there can be four elongated segments in total (per each associated spine 122). In some examples, the total area of each ablation electrode 161 or ablation electrode segment within a respective electrode region (discussed above with respect to FIG. 4A) is approximately 7.5 mm². In some examples, such as a configuration like that of FIG. 4A or FIG. 4B that implements the pairs of segments of FIG. 11, one pair of these electrode segments of a respective electrode/spine region 102 can be connected together by the generator 50 with the same charge (e.g., a positive charge), while another pair of these electrode segments of the same electrode/spine region 102 can be connected together by the generator 50 and having the same charge (e.g., a negative charge), such that a biphasic pulse field is provided between these two pairs of ablation electrode segments. In alternative examples, these adjacent elongated segments of each ablation electrode 161 (when in contact with tissue) can be disconnected from one other and separately connected to the generator 50 so that a biphasic pulse field can be provided between these two tissue contacting ablation electrode segments in a bipolar configuration to allow the flow of electrons between the ablation electrode segments.

In general, the ablation electrode 161 can include one or more serpentine longitudinally extending segments (in the illustrated example of FIG. 14, there are two, as mentioned above) with a total width W1 and a path width W2. The example in FIG. 14 includes six diagnostic electrodes 165 per ablation electrode 161. The end effector 100 may include one, two, three, four, five, or six diagnostic electrodes 165 per ablation electrode 161. Each diagnostic electrode is similarly sized with a width W3 that is approximately one and a half times the path width W2 of the ablation electrode 161 and approximately one half the total width W1 of an elongated segment of the ablation electrode 161. The diagnostic electrodes 165 are surrounded on two or three sides by a respective elongated segment of the ablation electrode 161.

FIG. 15 is an illustration of a second electrode configuration for the example end effector 100 including ablation electrodes 161 having elongated segments which are respectively segmented into small parallel stripes and diagnostic electrodes 165 positioned alongside the elongated ablation electrode segments. Each of the elongated segments has multiple electrically conductive stripes running parallel to each other to form an overall shape of a respective elongated segment that is similar to as shown in FIG. 14. Other ablation electrode shapes illustrated herein, and alternatives thereto as understood by a person skilled in the pertinent art may also be divided into multiple electrically conductive stripes running parallel to each other similar to as shown in FIG. 15. This configuration increases the total edge length of the ablation electrode to provide a different electric field profile to tissue during PFA than a similarly shaped ablation electrode that is solid (e.g., as shown in FIG. 14).

FIG. 16 is an illustration of a third example electrode configuration for the example end effector 100 including rectangular elongated segments of the ablation electrode 161 without diagnostic electrodes 165. The entire width W1 of the electrode segment is utilized for the ablation electrode 161 to maximize surface area of the ablation electrode 161. Diagnostic electrodes can be disposed elsewhere on the flexible membrane 130.

FIG. 17 is an illustration of a fourth example electrode configuration for the example end effector 100 including rectangular elongated segments of the ablation electrode 161 interrupted by diagnostic electrodes 165. Compared to the continuous rectangular electrode segments shown in FIG. 16, the segmented elongated ablation electrode segments can have greater flexibility for the membrane 130 to flex, at the expense of total ablation electrode area.

In accordance with all of the above-described examples, the end effector 100 preferably includes a total of two to eight ablation electrodes 161 per associated spine 122. The end effector 100 preferably includes exactly two, three, or four ablation electrodes (or electrode segment) per side of the membrane 130. Each ablation electrode 161 preferably overlaps a corresponding ablation electrode 161 on the opposite side of the membrane 130 so that the ablation electrodes are symmetric with respect to a plane defined by the membrane 130 when laid flat (e.g., pre-assembly to its umbrella shaped configuration). Fewer ablation electrodes can be accomplished by electrically connecting combinations of ablation electrodes within the end effector 100 or elsewhere within the catheter 14. Increasing the number of ablation electrodes 161 can be accomplished by splitting apart an ablation electrode 161 into two portions that are electrically insulated from each other in the catheter 14 and configured to be independently activated by the generator 50. For example, the cross-sectional line 4A-4A in FIG. 2A could serve as a dividing line in an example in which the ablation electrode 161A is segmented into two electrodes: a distal ablation electrode (portion of 161A to the left of line 4A-4A in FIG. 2A) and a proximal ablation electrode (portion of 161A to the right of line 4A-4A in FIG. 2A). Likewise, the ablation electrodes 161B, 161C, 161D can be divided in a similar manner.

In summary, ablation electrodes 161 can be paired in various pairing combinations to provide bipolar PFA electrical signals between treatment electrodes in a pair. Pairs can be activated simultaneously or sequentially in various combinations to achieve PFA of target tissue as understood by a person skilled in the pertinent art informed by the disclosure herein. The example treatment electrode configurations illustrated and described herein are non-limiting and numerous other treatment electrode configurations are possible. In each treatment electrode configuration, treatment electrodes can be paired following the same concepts as outlined in the foregoing disclosure.

The disclosed technology described herein can be further understood according to the following clauses:
Clause 1. An end effector of a medical probe, the end effector comprising: a plurality of spines extending radially outward from a longitudinal axis of the end effector in an expanded configuration; a membrane connected to the plurality of spines, the membrane comprising a distal end that surrounds the longitudinal axis and defining a volume with an open end, the membrane including a first surface and a second surface opposite the first surface; a first flexible circuit disposed on the first surface of the membrane proximate each of the plurality of spines and extending around the longitudinal axis; a second flexible circuit disposed on the second surface of the membrane proximate each of the plurality of spines and extending around the longitudinal axis; a plurality of first pairs of electrodes disposed on the first flexible circuit proximal to one another and a spine of the plurality of spines; and a plurality of second pairs of electrodes disposed on the second flexible circuit proximal to one another and a spine of the plurality of spines such that the first and second pairs of electrodes, with the membrane between the first and second pairs of electrodes, define a mirror image configuration.
Clause 2. The end effector of clause 1, the plurality of spines being radially disposed about the longitudinal axis.
Clause 3. The end effector of any one of clauses 1-2, each spine comprising a proximal end fixed to a probe shaft and a free distal end, the distal end of the membrane extending beyond the free distal end of each spine.
Clause 4. The end effector of any one of clauses 1-2, each spine comprising a proximal end fixed to a first probe shaft and a distal end fixed to a second probe shaft.
Clause 5. The end effector of any one of clauses 1-4, the membrane being biased to the expanded configuration by the plurality of spines.
Clause 6. The end effector of any one of clauses 1-5, the membrane comprising a generally semi-spherical, semi-conical shape, or polygonal pyramidal shape in the expanded configuration that defines the volume and further comprising a polymer material.
Clause 7. The end effector of any one of clauses 1-6, the end effector being moveable between a collapsed configuration, in which the plurality of spines and the membrane are disposed generally along the longitudinal axis, and the expanded configuration.
Clause 8. The end effector of any one of clauses 1-7, the first surface facing away from the longitudinal axis and the second surface facing towards the longitudinal axis.
Clause 9. The end effector of any one of clauses 1-8, the first flexible circuit and the second flexible circuit being spaced apart from the plurality of spines along a vertical axis, each first pair of electrodes being respectively electrically connected together to define a single first electrode of a plurality of first electrodes on the first surface and each second pair of electrodes being respectively electrically connected together to define a single second electrode of a plurality of second electrodes on the second surface.
Clause 10. The end effector of clause 9, a portion of the membrane being disposed between the plurality of spines and the first flexible circuit and between the plurality of spines and the second flexible circuit.
Clause 11. The end effector of any one of clauses 1-10, further comprising a plurality of voids defined through the membrane, the first flexible circuit, and the second flexible circuit between adjacent spines of the plurality of spines.
Clause 12. The end effector of clause 11, each void comprising a closed perimeter defined by the membrane.
Clause 13. The end effector of any one of clauses 1-12, the membrane comprising a flexible biocompatible polymer material.
Clause 14. The end effector of any one of clauses 1-13, the membrane comprising a plurality of layers, each layer surrounding the longitudinal axis, with at least one of the layers comprising an insulative material.
Clause 15. The end effector of clause 14, the plurality of layers of each leaf comprising at least one layer comprising a dielectric material.
Clause 16. The end effector of any one of clauses 1-15, the first flexible circuit comprising a plurality of first flexible substrates that are circumferentially spaced around the longitudinal axis, each first electrode being disposed on a respective first flexible substrate.
Clause 17. The end effector of any one of clauses 1-16, a number of spines of the plurality of spines being greater than a number of the first pairs of electrodes.
Clause 18. The end effector of any one of clauses 1-17, each first pair of electrodes being flush with an outer surface of the membrane.
Clause 19. The end effector of any one of clauses 1-17, each first pair of electrodes protruding from an outer surface of the membrane.
Clause 20. The end effector of any one of clauses 1-19, the plurality of first pairs of electrodes and the plurality of second pairs of electrodes being ablation electrodes, and further comprising one or more diagnostic electrodes.
Clause 21. The end effector of any one of clauses 1-20, each electrode of the first and second pairs of electrodes comprising an elongated segment comprising a plurality of conductive stripes running parallel to each other to form an overall shape of the elongated segment.
Clause 22. A medical system comprising: a medical probe comprising an elongated probe body and an end effector connected to a distal end of the elongated probe body, the elongated probe body and the end effector extending along a longitudinal axis, the end effector comprising: a plurality of spines connected to the elongated probe body and extending radially outward from the longitudinal axis of the end effector in an expanded configuration; a membrane connected to the plurality of spines, the membrane comprising a distal end that surrounds the longitudinal axis and defining a volume with an open end, the membrane including a first surface and a second surface opposite the first surface; a first flexible circuit disposed on the first surface of the membrane proximate each of the plurality of spines and extending around the longitudinal axis; a second flexible circuit disposed on the second surface of the membrane proximate each of the plurality of spines and extending around the longitudinal axis; a plurality of first pairs of ablation electrodes disposed on the first flexible circuit and proximal to one another and a spine of the plurality of spine; and a plurality of second pairs of electrodes disposed on the second flexible circuit proximal to one another and a spine of the plurality of spines such that the first and second pairs of electrodes, with the membrane between the first and second pairs of electrodes, define a mirror image configuration; and an ablation generator configured to provide ablation pulses to the plurality of first pairs of electrodes and the plurality of second pairs of electrodes.
Clause 23. The medical system of clause 22, the plurality of first pairs of ablation electrodes comprising a first ablation electrode and a second ablation electrode disposed proximal to one another, and the plurality of second pairs of ablation electrodes comprising a third ablation electrode and a fourth ablation electrode disposed proximal to one another and one of the first pair of ablation electrodes.
Clause 24. The medical system of clause 23, the first ablation electrode and the second ablation electrode of each first pair being disposed on opposing lateral sides, relative to the respective spine, and the third ablation electrode and the fourth ablation electrode of each second pair being disposed on opposing lateral sides, relative to the respective spine.
Clause 25. The medical system of any one of clauses 23-24, the ablation generator being configured to provide ablation pulses between the first ablation electrode and the fourth ablation electrode of each respective first and second pairs of electrodes.
Clause 26. The medical system of any one of clauses 23-24, the ablation generator being configured to provide ablation pulses between the first ablation electrode and the third ablation electrode of each respective first and second pairs of electrodes.
Clause 27. The medical system of any one of clauses 23-24, the ablation generator being configured to provide ablation pulses between the first ablation electrode and the second ablation electrode of each respective first pair of electrodes.
Clause 28. The medical system of clause 23, each set of proximal first, second, third, and fourth ablation electrodes defining respective electrode and spine regions, the ablation generator being configured to provide ablation pulses between different electrode and spine regions.
Clause 28. A method of manufacturing an end effector for a medical probe, the method comprising: forming a plurality of spines extending away from a longitudinal axis; disposing a first flexible circuit on a first side of the plurality of spines, the first flexible circuit comprising a first flexible substrate and a first pair of electrodes; disposing a second flexible circuit on a second side of the plurality of spines, the second flexible circuit comprising a second flexible substrate and a second pair of electrodes; placing a first sheet of insulative material in contact with the first flexible circuit, the first sheet surrounding the longitudinal axis; placing a second sheet of insulative material in contact with the second flexible circuit, the second sheet surrounding the longitudinal axis and defining a volume with an open end; and molding the first sheet and the second sheet to envelop the first flexible circuit and the second flexible circuit.
Clause 29. The method of clause 28, further comprising: prior to molding the first sheet and the second sheet, placing a third sheet of insulative material between the first flexible circuit and the second flexible circuit and in contact with the plurality of spines; and molding the third sheet such that the first flexible circuit and the second flexible circuit is spaced apart from the plurality of spines.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

To the extent that any materials incorporated by reference herein contain similar terms but differ in definition or description, it will be appreciated that the definitions or descriptions provided herein are to be used in understanding the technology disclosed herein.

## Claims

1. An end effector of a medical probe, the end effector comprising:
a plurality of spines extending radially outward from a longitudinal axis of the end effector in an expanded configuration;
a membrane connected to the plurality of spines, the membrane comprising a distal end that surrounds the longitudinal axis and defining a volume with an open end, the membrane including a first surface and a second surface opposite the first surface;
a first flexible circuit disposed on the first surface of the membrane proximate each of the plurality of spines and extending around the longitudinal axis;
a second flexible circuit disposed on the second surface of the membrane proximate each of the plurality of spines and extending around the longitudinal axis;
a plurality of first pairs of electrodes disposed on the first flexible circuit proximal to one another and a spine of the plurality of spines; and
a plurality of second pairs of electrodes disposed on the second flexible circuit proximal to one another and a spine of the plurality of spines such that the first and second pairs of electrodes, with the membrane between the first and second pairs of electrodes, define a mirror image configuration.

2. The end effector of claim 1, the plurality of spines being radially disposed about the longitudinal axis.

3. The end effector of claim 1 or claim 2, each spine comprising i) a proximal end fixed to a probe shaft and a free distal end, the distal end of the membrane extending beyond the free distal end of each spine, or ii) a proximal end fixed to a first probe shaft and a distal end fixed to a second probe shaft.

4. The end effector of any preceding claim, the membrane being biased to the expanded configuration by the plurality of spines.

5. The end effector of any preceding claim, the membrane comprising a generally semi-spherical, semi-conical shape, or polygonal pyramidal shape in the expanded configuration that defines the volume and further comprising a polymer material.

6. The end effector of any preceding claim, the end effector being moveable between a collapsed configuration, in which the plurality of spines and the membrane are disposed generally along the longitudinal axis, and the expanded configuration.

7. The end effector of any preceding claim, the first surface facing away from the longitudinal axis and the second surface facing towards the longitudinal axis.

8. The end effector of any preceding claim, the first flexible circuit and the second flexible circuit being spaced apart from the plurality of spines along a vertical axis, each first pair of electrodes being respectively electrically connected together to define a single first electrode of a plurality of first electrodes on the first surface and each second pair of electrodes being respectively electrically connected together to define a single second electrode of a plurality of second electrodes on the second surface, optionally a portion of the membrane being disposed between the plurality of spines and the first flexible circuit and between the plurality of spines and the second flexible circuit.

9. The end effector of any preceding claim 1, further comprising a plurality of voids defined through the membrane, the first flexible circuit, and the second flexible circuit between adjacent spines of the plurality of spines.

10. The end effector of any preceding claim, the membrane comprising a plurality of layers, each layer surrounding the longitudinal axis, with at least one of the layers comprising an insulative material, optionally the plurality of layers of each leaf comprising at least one layer comprising a dielectric material.

11. The end effector of any preceding claim, the first flexible circuit comprising a plurality of first flexible substrates that are circumferentially spaced around the longitudinal axis, each first electrode being disposed on a respective first flexible substrate.

12. The end effector of any preceding claim, a number of spines of the plurality of spines being greater than a number of the first pairs of electrodes.

13. The end effector of any preceding claim, each first pair of electrodes (i) being flush with an outer surface of the membrane or (ii) protruding from an outer surface of the membrane.

14. The end effector of any preceding claim, each electrode of the first and second pairs of electrodes comprising an elongated segment comprising a plurality of conductive stripes running parallel to each other to form an overall shape of the elongated segment.

15. A medical system comprising:
a medical probe comprising an elongated probe body and an end effector connected to a distal end of the elongated probe body, the elongated probe body and the end effector extending along a longitudinal axis, the end effector comprising:
a plurality of spines connected to the elongated probe body and extending radially outward from the longitudinal axis of the end effector in an expanded configuration;
a membrane connected to the plurality of spines, the membrane comprising a distal end that surrounds the longitudinal axis and defining a volume with an open end, the membrane including a first surface and a second surface opposite the first surface;
a first flexible circuit disposed on the first surface of the membrane proximate each of the plurality of spines and extending around the longitudinal axis;
a second flexible circuit disposed on the second surface of the membrane proximate each of the plurality of spines and extending around the longitudinal axis;
a plurality of first pairs of ablation electrodes disposed on the first flexible circuit and proximal to one another and a spine of the plurality of spine; and
a plurality of second pairs of electrodes disposed on the second flexible circuit proximal to one another and a spine of the plurality of spines such that the first and second pairs of electrodes, with the membrane between the first and second pairs of electrodes, define a mirror image configuration; and
an ablation generator configured to provide ablation pulses to the plurality of first pairs of electrodes and the plurality of second pairs of electrodes.

16. A method of manufacturing an end effector for a medical probe, the method comprising:
forming a plurality of spines extending away from a longitudinal axis;
disposing a first flexible circuit on a first side of the plurality of spines, the first flexible circuit comprising a first flexible substrate and a first pair of electrodes;
disposing a second flexible circuit on a second side of the plurality of spines, the second flexible circuit comprising a second flexible substrate and a second pair of electrodes;
placing a first sheet of insulative material in contact with the first flexible circuit, the first sheet surrounding the longitudinal axis;
placing a second sheet of insulative material in contact with the second flexible circuit, the second sheet surrounding the longitudinal axis and defining a volume with an open end; and
molding the first sheet and the second sheet to envelop the first flexible circuit and the second flexible circuit.
